(19) Europäisches Patentamt
European Patent Office
Office européen des brevets

(11) Publication number: 0 010 852
B1

(12) EUROPEAN PATENT SPECIFICATION

(45) Date of publication of patent specification: 08.09.82

(21) Application number: 79301968.8

(22) Date of filing: 21.09.79

(51) Int. Cl.³: C 07 D 513/04, C 07 D 233/06, C 07 D 233/42, C 07 C 91/08, C 07 C 93/04 //(C07D513/04, 277/00, 235/00)

# (54) Process for the preparation of tetramisole.

(30) Priority: 06.11.78 US 958221
02.08.79 US 63278

(43) Date of publication of application:
14.05.80 Bulletin 80/10

(45) Publication of the grant of the patent:
08.09.82 Bulletin 82/36

(84) Designated Contracting States:
AT BE CH DE FR GB IT LU NL SE

(56) References cited:
DE - A - 2 829 822
US - A - 3 726 894
US - A - 3 845 070
US - A - 3 873 560
US - A - 4 090 025

(73) Proprietor: AMERICAN CYANAMID COMPANY
Berdan Avenue
Wayne New Jersey 06904 (US)

(72) Inventor: Raghu, Sivaraman
45 Lois Street
Norwalk, Connecticut (US)

(74) Representative: Allam, Peter Clerk et al,
LLOYD WISE, TREGEAR & CO. Norman House
105-109 Strand
London WC2R 0AE (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for the preparation of tetramisole

The present invention relates to a process for the preparation of tetramisole either as a salt or in free base form.

The synthesis of tetramisole or racemic 2,3,5,6-tetrahydro-6-phenylimidazo[2,1-b]thiazole and its pharmaceutically acceptable addition salts is of considerable commercial interest because of the anthelminthic activity of such compounds. The enantiomers of this compound are well known and the laevorotatory isomer is extremely well suited to such uses, as discussed in U.S. Patent 3,463,786.

As a consequence of such activity, various syntheses are known. In this connection, there are cited Raeymaekers et al., *J. Med. Chem. 9,* 545 (1966); Bakeline et al., *Aust J. Chem. 21,* 1557 (1968); Roy U.S. Patent 3,855,234; McMemin U.S. Patent 3,845,070; and Spicer U.S. Patent 3,726,894.

In the McMemin U.S. Patent No. 3,845,070 tetramisole is prepared via a route in which N-(2-amino-2-phenylethyl)-2-hydroxyethylamine is made as an intermediate. Although this diamine is one of the intermediates which may be prepared in the process of the present invention, the present process uses different procedures both to prepare the diamine and thereafter to convert it to tetramisole.

The process for the preparation of tetramisole in accordance with the present invention comprises the steps of:

(a) reacting an imidoyl halide having the formula:

$$X—C(R_1)=N—CH(Ar)—CH_2—X$$

wherein Ar is phenyl, $R_1$ is hydrogen, lower alkyl, or phenyl or lower alkyl-substituted phenyl, and X is halo, with hydroxyethylamine or alkoxyethylamine having the formula:

$$R_2O—(CH_2)_2—NH_2$$

wherein $R_2$ is hydrogen or lower alkyl to obtain an amidine hydrohalide having the formula

$$R_2O—CH_2—CH_2—NH—C(R_1)=N—CH(Ar)—CH_2X.HX;$$

(b) reacting the latter amidine hydrohalide at a temperature ranging from –20°C to 50°C with either an inorganic base, hydroxyethylamine or alkoxyethylamine having the formula:

$$H_2N—CH_2—CH_2—OR_2$$

in an inert medium to obtain an imidazoline having the formula:

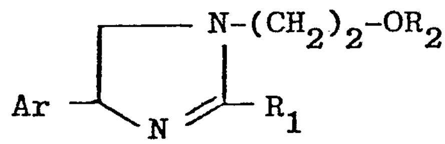

where Ar, $R_1$ and $R_2$ are as defined above;

(c) hydrolyzing the latter imidazoline at a temperature ranging from 25°C to 150°C to cleave the ring to thereby obtain an amidoamine having the formula:

$$R_1CONH—CH(Ar)—CH_2—NH—CH_2—CH_2—OR_2$$

where Ar, $R_1$ and $R_2$ are as defined above;

(d) further reacting the latter amidoamine with a suitable inorganic base or protic source to obtain a diamine having the formula:

$$NH_2—CH(Ar)—CH_2—NH—CH_2—CH_2—OR_2$$

wherein Ar, $R_1$, and $R_2$ are as above defined;

(e) reacting the latter diamine with carbon disulfide to provide a dithiocarbamate having the tautomeric formula:

$$^{\ominus}SC(S)—NH—CH(Ar)—CH_2—^{\oplus}NH_2(CH_2)_2—OR_2;$$

(f) heating the latter dithiocarbamate to produce a thione having the formula:

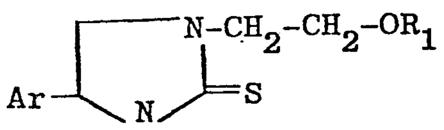

(g) reacting the latter thione with the acid having the formula: HA to provide a tetramisole salt having the formula:

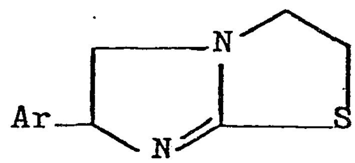

HA

wherein Ar, $R_1$ and $R_2$ are as defined above, and A is an anion of a pharmaceutically acceptable acid, and if desired

(h) neutralizing the latter to obtain tetramisole in free base form, of the formula:

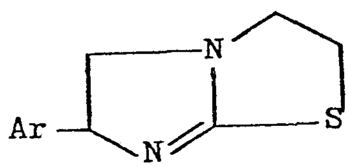

The imidoyl halide of the formula:

$$X—C(R_1)=N—CH(Ar)—CH_2—X$$

wherein Ar, $R_1$ and X are as defined above which is used as the starting material in the present process may itself be prepared by reacting styrene with a halogen and a nitrile to provide the imidoyl halide by mesne reaction.

The desirable halogens for use in practicing the present invention include chlorine, bromine, and iodine. Because of the ease of dispersion; the reaction velocity, and economy, chlorine is a preferred halogen in certain embodiments of the present invention.

The nitrile has the formula $R_1—C≡N$ (II) where $R_1$ is hydrogen, lower alkyl, phenyl or lower alkyl-substituted phenyl. A preferred nitrile is benzonitrile.

Useful lower alkyl nitriles are those containing from two to about seven carbon atoms per molecule. The use of longer chain or unsaturated nitriles can complicate the process and increase the cost of the raw materials without any concomitant benefit. It is especially desirable to utilize the lower nitriles, such as acetonitrile and, as taught above, benzonitrile in certain preferred embodiments of the invention.

This reaction between styrene, halogen and nitrile is carried out at temperatures ranging from slightly above room temperature to relatively lower temperatures. The desirable temperature range for use in connection with this step is from about –20°C to about 30°C. Moreover, the temperature is lowered to freezing or below and then increased to room temperature or somewhat higher after all of the reactants have been combined. The reaction can be carried out in the presence of an inert vehicle, such as alkyl or aromatic hydrocarbons or halogenated hydrocarbons.

The quantities of reactants used for this preliminary step can range from stoichiometric up to an excess of the halogen and the nitrile. When the nitrile is present in excess, it can act as a vehicle to assist in moderation and control of the reaction, and this is done in certain preferred embodiments. Generally, it has been found desirable to admix the styrene and the nitrile and then to introduce the halogen into the mixture. It is also possible to add one or the other of the liquid reactants over a period of time while the halogen is being introduced into the mixture.

The time for this step of the process will vary according to the temperature and the particular reactants. Generally, at temperatures at or near 0°C, times of one to four hours give good results. The mixture can then be permitted to rise in temperature, as taught above, and held for another one to four hours.

This reaction of nitrile, styrene and halogen provides imidoyl halide compounds having the formula

$$X—C(R_1)=N—CH(Ar)—CH_2—X \qquad (III)$$

where $R_1$, Ar and X have the meanings set forth above. In certain preferred embodiments, $R_1$ is hydrogen, phenyl or an alkyl group having from one to four carbon atoms, more particularly methyl or ethyl.

According to the present invention, the imidoyl halide so formed is treated with specific amines to provide an amidine hydrohalide. The amide is an alkoxyethylamine or the corresponding hydroxyamine, such amines having the formula

$$H_2N—CH_2—CH_2—OR_2 \qquad (IV)$$

where $R_2$ is hydrogen or a lower alkyl group, desirably those containing from one to four carbon atoms, more especially methyl or ethyl and in certain preferred embodiments methoxyethylamine is especially preferred.

The amine IV can be added directly to the reaction mixture of the preceding step. The temperature for this stage of the process can range from −20°C to 30°C. It is generally desirable to add the amine at a temperature of about 0°C to 5°C or below. The temperature of the reaction mixture is desirably maintained below 20°C with cooling during addition of the amine. Thereafter, the temperature can be allowed to rise slowly to achieve improved reaction completeness. The reaction with amine can be carried out in the presence of a vehicle. When excess nitrile has been used in certain preferred embodiments of the invention to produce the imidoyl halide, the nitrile itself will act as a reaction vehicle. Any non-nucleophilic solvent inert to hydrogenation, such as saturated hydrocarbons, halogenated hydrocarbons, ethers, and the like, can be used.

Addition of the amine is carried out over a period of time sufficient to permit reaction of the newly added material. In small-scale preparations, times of from about 30 minutes to four hours have been found to be desirable. The quantity of amine used is stoichiometric for formation of the amidine hydrohalide or slightly in excess of stoichiometric to provide good reaction completeness.

The product produced according to this stage of the reaction is an amidine hydrohalide having the formula

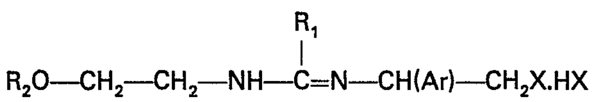

(V),

where Ar, $R_1$, $R_2$ and X have the meanings set forth above. Any excess nitrile can be removed from the product at this point by conventional techniques, such as distillation, vacuum stripping and the like. If a vehicle other than the nitrile is used, it can also be removed from the amidine at this point. The amidine hydrohalide itself can be purified, if desired, and recovered in a purified form. However, in a preferred embodiment of the invention, the amidine can be further treated directly before or after removal of excess nitrile.

The amidine hydrohalide is next treated with an inorganic base or excess hydroxyethylamine or alkoxyethylamine of the formula

$$H_2N—CH_2—CH_2—OR_2$$

to deprotonate it and thereby form an imidazoline ring having the structure

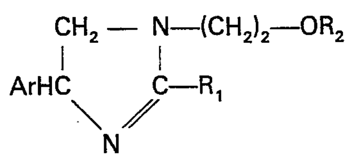

(VI)

wherein Ar, $R_1$ and $R_2$ have the meanings already given. The cyclization of the amidine is carried out at temperatures which provide a satisfactory rate of reaction while avoiding conditions which would be detrimental to the product. These temperatures are in the range from −20°C to 50°C. The cyclization is carried out in the presence of an inert reaction vehicle such as excess nitrile from the imidoyl halide step or in hydrocarbons, including toluene, xylenes, and the like, halogenated hydrocarbons, lower halogenated hydrocarbons having from one to three carbon atoms, such as methylene chloride, ethylene dichloride and the like being preferred. It has also been found possible according to the present invention to use excess amine with the imidoyl halide to go directly to imidazoline VI.

The next step of the process is treatment of the imidazoline VI with a base or protic source to hydrolyze the cyclic compound to an amidoamine (VII) having the formula

$$R_1CONH—CH(Ar)—CH_2—NH—(CH_2)_2—OR_2.$$

$R_1$, $R_2$ and Ar have the meanings set forth above.

The ring cleavage hydrolysis is carried out at a sufficient temperature to provide reasonable reaction velocity and below the temperature at which undesired further cleavage or side reactions occur. A temperature in the range of from 25° to 150°C is utilized. The hydrolytic agent used can be water, a base such as an alkali metal hydroxide, or an acid, such as a mineral acid. For reasons of yield and economy, preferred materials are the alkali metal hydroxides, such as aqueous sodium hydroxide, and aqueous mineral acids, such as sulfuric acid and hydrochloric acid. Among the acids, a 10 to 50% aqueous hydrochloric acid is preferred, and among the bases, a 10 to 40% aqueous sodium hydroxide is a preferred reagent.

Amidoamine VII so produced may, if desired, be separated from the reaction mixture by conventional methods, such as solvent extraction before the next step of the process. Preferred solvents for use in such solvent extraction include the lower chlorinated hydrocarbons, including mono- and polychloro alkyl groups, having from one to three carbon atoms, with methylene chloride being a preferred solvent.

The amidoamine is then treated with a base or protic source to provide the corresponding diamine having the formula

$$NH_2—CH(Ar)—CH_2—NH—(CH_2)_2—OR_2 \qquad \text{(VIII)},$$

wherein Ar and $R_2$ are as already defined. The conditions used to produce the diamine and the preferred reactants are the same as stated for ring cleavage.

The diamine is now reacted with carbon disulfide to provide dithiocarbamate intermediate, represented by the tautomeric formula

$$^{\ominus}SC(S)—NH—CH(Ar)CH_2—^{\oplus}NH(CH_2)_2—OR_2 \qquad \text{(IX)}$$

followed by cyclization with heat to produce 1-substituted-4-arylimidazolidin-2-thione having the formula

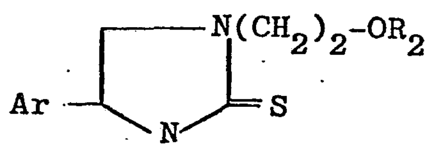

(X)

The thione so produced is then treated with an acid having a pharmaceutically acceptable anion to provide imidazothiazole:

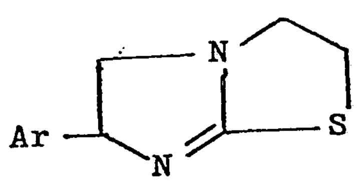

HA (XI)

It will be recognised that these are the pharmaceutically acceptable salts of *d,l*-6-phenyl-2,3,5,6-tetrahydroimidazo[2,1-b]thiazole or tetramisole. Such acid compounds can be neutralized with a base to provide the free tetramisole, when this is desired.

The dithiocarbamate is prepared by reacting diamine VIII with carbon disulfide at temperatures of from −10° to 40°C. It is generally desirable to use from a 50 to 100% stoichiometric excess of carbon disulfide. This reaction step is desirably carried out in the presence of an inert vehicle such as one or more of the hydrocarbons or chlorinated hydrocarbons. Preferred hydrocarbons include lower alkyl, cycloalkyl, and aromatic materials such as benzene, toluene, xylenes, and the like, liquid aliphatic hydrocarbons having from five to 12 carbon atoms, such as hexane, isooctane, heptane, and the like, and liquid cycloaliphatic materials such as cyclohexane, cyclooctane, and the like; and chlorinated

hydrocarbons include the polyhalogenated lower aliphatic materials, a preferred vehicle being tetrachloroethane.

The reaction time ranges from about 30 minutes to about four hours in certain desirable embodiments of the invention. The resulting dithio compound IX is cyclized by heating at 80° to 150°C. The ring closure to provide thione X is carried out for from about two to about 20 hours. Production of the pharmaceutically acceptable salt of the tetramisole is then effected on the recovered thione by acid treatment to close the thiaza ring.

It will be understood from the present disclosure that the various intermediates can be recovered and purified as desired by conventional techniques such as extraction, solvent evaporation, water washing, and combinations of these conventional procedures. Further, the various steps can be carried out under subatmospheric or superatmospheric pressure. Unless superatmospheric pressure is desirable because of the volatility of a solvent or reactant, it is generally preferred to conduct all of the steps under atmospheric pressure. This provides further economy in not requiring special pressure vessels and handling techniques in commercial production.

The following Examples are given to illustrate the invention.

## Example 1

*Preparation of 1-(2-Methoxyethyl)-2-methyl-4-phenyl-2-imidazoline*

A mixture of 41.6 g styrene and 131.2 g acetonitrile is cooled to 0°C, and 28.4 g chlorine is bubbled through the mixture at a temperature of 0° to 5°C during 75 minutes. The temperature is thereupon maintained at the same level, and 33 g 2-methoxyethylamine is added dropwise.

The cooling bath used to maintain the low temperature is removed, and the temperature of the solution is allowed to rise gradually to 39°C without any external heating. The mixture is then maintained at about 55°C for 75 minutes. Thereafter, the acetonitrile is distilled off.

To the residue is added 200 ml 1N aqueous hydrochloric acid and 100 ml methylene chloride, and the mixture is allowed to separate into an aqueous phase and an organic phase. The aqueous layer is separated and rendered basic with sufficient 20% aqueous sodium hydroxide solution in the presence of 200 ml methylene chloride.

The resulting methylene chloride layer is separated, washed and dried, and the solvent is removed to provide 36 g of the imidazoline in the form of a viscous yellow oil. This product is identified as the imidazoline by infrared (IR) and proton magnetic resonance (PMR) spectroscopy.

## Example 2

*Preparation of 1-(2-Hydroxyethyl)-2-methyl-4-phenyl-2-imidazoline*

A stirred mixture of 83 g styrene and 262 g acetonitrile is cooled to 0°C, and 57 g of gaseous chlorine is bubbled through the mixture during one hour, while the temperature is maintained between 0° to 5°C. Thereafter, 54 g ethanolamine is added during 40 minutes, with the temperature being in the range of 0° to 5°C.

The cooling bath is then removed, and the temperature is allowed to rise to 35°C. The reaction mixture is thereafter maintained at 50°C for one hour by application of external heating. The acetonitrile is then removed by distillation.

The material remaining after distillation is a viscous semi-solid residue, to which is added 200 ml 1N aqueous hydrochloric acid. The material separates into two layers. The aqueous layer is separated from the methylene dischloride layer and made basic with 20% aqueous sodium hydroxide solution. The basic aqueous solution is extracted with three 100 ml portions of methylene chloride.

The combined organic extracts are washed and dried, and the solvent is evaporated to provide 66 g of a viscous yellow oil. IR and PMR spectroscopy identify the imidazoline.

## Example 3

*Preparation of 1-(2-Hydroxyethyl)-2,4-diphenyl-2-imidazoline*

A mixture of 20.8 g styrene and 103 g benzonitrile is cooled to 0°C and maintained at that temperature while 14 g gaseous chlorine is bubbled through the mixture during 30 minutes. Thereafter, 13.5 g ethanolamine is added during 15 minutes, while the temperature is maintained at 0° to 5°C.

The cooling bath is then removed and the mixture is slowly heated to 45°C and maintained at this temperature two hours. After the mixture is cooled, 100 ml 1N aqueous hydrochloric acid is added to form two layers. The organic layer is separated from the aqueous layer, is made basic with 20% aqueous sodium hydroxide solution, and extracted twice with 100 ml portion of methylene chloride.

The combined methylene chloride extracts are washed and dried, and the solvent is removed by evaporation to provide 14 g of a viscous oil product. This is identified as the imidazoline by IR and PMR spectroscopy.

## Example 4

*Preparation of N-(2-Amino-2-phenylethyl)-2-methoxyethylamine*

Ten grams of the methoxyethylmethylphenylimidazoline product of Example 1 is refluxed with 50 ml 30% aqueous sodium hydroxide solution for 24 hours. The mixture is then permitted to separate into

two phases, and the aqueous layer is extracted with methylene chloride. The extract is concentrated and the resulting oil, which is identified as the intermediate amide, is refluxed with 20% aqueous sulfuric acid for six hours.

The resulting mixture is extracted with methylene chloride to remove impurities, and the aqueous solution is made basic with 20% aqueous sodium hydroxide solution and extracted with methylene chloride. The mixture is permitted to phase separate and the organic layer is separated, washed, and dried. The solvent is removed to obtain the methoxyethylamine as an oil.

The oil so obtained is distilled under reduced pressure to collect a purer product in the form of a pale yellow oil with a boiling point of 110°—115°C at 0.1—0.2 mm Hg. The product identity is confirmed by IR and PMR spectroscopy.

Example 5

*Preparation of N-(2-Amino-2-phenylethyl)-2-hydroxyethylamine*

The hydroxyethylmethylphenylimidazoline produced in Example 2 in the amount of 30 g and 60 g potassium hydroxide, in 150 ml ethanol and 40 ml water, are refluxed for 24 hours. The solution is then concentrated by removal of the ethanol, and the residue is taken up in 50 ml of water.

The resulting aqueous liquid is then extracted thrice with 100 ml portions of methylene chloride. The extracts are combined, washed, and dried.

The solvent is removed to provide the hydroxyethylamine, which is a yellow oil. IR and PMR analyses confirm its structure.

Example 6

*Preparation of 1-(2-Hydroxyethyl)-4-phenylimidazoline-2-thione*

The methoxyethylamine product of Example 4 in the amount of 4.5 g is dissolved in 20 ml of tetrachloroethane, and is stirred with 2 ml carbon disulfide at room temperature for one hour. The resulting slurry is then slowly heated to 120°C and maintained at that temperature for four hours.

After heating, the mixture is cooled to room temperature and stirred overnight. The tetrachloroethane is then distilled off under reduced pressure to leave a residual semi-solid. This material is identified as the thione by IR and PMR spectroscopy.

Example 7

*Preparation of 1-(2-Methoxyethyl)-4-phenylimidazolidin-2-thione*

The ethylamine product of Example 4 in the amount of 6.85 g is dissolved in 20 ml xylene, and this is then stirred with 3 ml carbon disulfide at room temperature for two hours. The resulting slurry is then slowly heated to 130°C and maintainerd at this temperature for four hours.

The xylene solvent is then distilled off under reduced pressure, and the residue is identified as the thione by IR and PMR spectroscopic techniques.

Example 8

*Preparation of DL-Tetramisole*

The phenylimidazolidinthione product of Example 6 in the amount of 4.3 g is suspended in 50 ml concentrated aqueous hydrochloric acid. The mixture is slowly heated to 70°C while agitated with a magnetic stirrer. The mixture is maintained at this temperature for ten hours, cooled, and stirred at room temperature overnight.

The solution is diluted in 50 ml water and impurities are extracted with two 30 ml methylene chloride treatments. The aqueous layer is made basic with ammonium hydroxide and extracted thrice with 50 ml portions of methylene chloride. The methylene chloride extracts are washed and dried.

The remaining methylene chloride solvent is removed to provide an oil which crystallizes. This crystalline product is identified by IR and PMR spectroscopy as DL-tetramisole [(±)-6-phenyl-2,3,5,6-tetrahydroimidazo[2,1-b]thioazole].

Example 9

*Preparation of DL-Tetramisole*

The 1-2(methoxyethyl)-4-phenylimidazolidin-2-thione product of Example 7 in the amount of 0.78 g is suspended in 50 ml concentrated aqueous hydrochloric acid and maintained with stirring at 70°—75°C for four hours. The solution is then cooled to room temperature and diluted with 50 ml water.

Some impurities are extracted with methylene chloride. The aqueous layer is made basic with ammonium hydroxide and extracted twice with 60 ml portions of methylene chloride. The extracts are combined, washed and dried.

The solvent is removed by distillation to provide an oil which crystallizes. The crystallized material is identified as DL-tetramisole by IR and PMR spectroscopy.

Example 10

*Preparation of N-Substituted Hydroxyethylamine*

The 1-(2-hydroxyethyl)-2-methyl-4-phenyl-2-imidazoline product of Example 2 in the amount of 5 g is refluxed with 20 ml water for two hours. The solution is cooled, saturated with sodium chloride, and extracted with three 50 ml portions of methylene chloride. The extracts are combined and dried over sodium sulfate, and the solvent is removed to provide an oil which becomes semi-solid on standing.

Addition of 10 ml acetone and cooling give a solid which is filtered and dried. The solid has a melting point of 133°—136°C. Trituration with acetone followed by filtration provide 31 g of N-(2-acetaminophenylethyl)-2-hydroxyethylamine, which has a melting point of 140°—143°C after air drying.

**Claims**

1. A process for the preparation of tetramisole which comprises the steps of:

(a) reacting an imidoyl halide having the formula:

$$X—C(R_1)=N—CH(Ar)—CH_2—X$$

wherein Ar is phenyl, $R_1$ is hydrogen, lower alkyl, or phenyl or lower alkyl-substituted phenyl, and X is halo, with hydroxyethylamine or alkoxyethylamine having the formula:

$$R_2O—(CH_2)_2—NH_2$$

wherein $R_2$ is hydrogen or lower alkyl to obtain an amidine hydrohalide having the formula:

$$R_2O—CH_2—CH_2—NH—C(R_1)=N—CH(Ar)—CH_2X.HX;$$

(b) reacting the latter amidine hydrohalide at a temperature ranging from −20°C to 50°C with either an inorganic base, hydroxyethylamine or alkoxyethylamine having the formula:

$$H_2N—CH_2—CH_2—OR_2$$

in an inert medium to obtain an imidazoline having the formula:

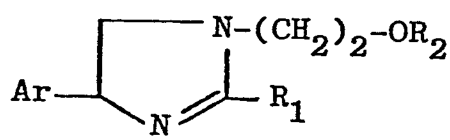

where Ar, $R_1$ and $R_2$ are as defined above;

(c) hydrolyzing the latter imidazoline at a temperature ranging from 25°C to 150°C to cleave the ring to thereby obtain an amidoamine having the formula:

$$R_1CONH—CH(Ar)—CH_2—NH—CH_2—CH_2—OR_2$$

where Ar, $R_1$ and $R_2$ are as defined above;

(d) further reacting the latter amidoamine with a suitable inorganic base or protic source to obtain a diamine having the formula:

$$NH_2—CH(Ar)—CH_2—NH—CH_2—CH_2—OR_2$$

wherein Ar, $R_1$ and $R_2$ are as above defined;

(e) reacting the latter diamine with carbon disulfide to provide a dithiocarbamate having the tautomeric formula:

$$^{\ominus}SC(S)—NH—CH(Ar)—CH_2—{}^{\oplus}NH_2(CH_2)_2—OR_2;$$

(f) heating the latter dithiocarbamate to produce a thione having the formula:

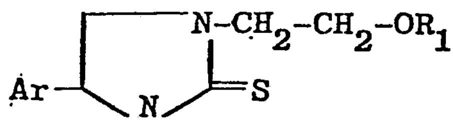

;

(g) reacting the latter thione with the acid having the formula: HA to provide a tetramisole salt having the formula:

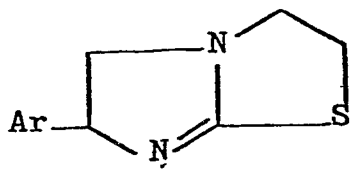

HA

wherein Ar, $R_1$ and $R_2$ are as defined above, and A is an anion of a pharmaceutically acceptable acid, and if desired

(h) neutralizing the latter to obtain tetramisole in free base form, of the formula:

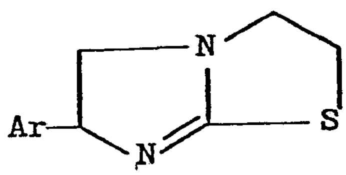

2. A process according to Claim 1, wherein step (a) is conducted by adding said hydroxyethylamine or alkoxyethylamine to said imidoyl halide over a period of from one-half to four hours whilst maintaining the temperature during said amine addition in the range below 20°C, whereafter the reaction is completed at a higher temperature.

3. A process according to Claim 1 or Claim 2, wherein said inert medium used in step (b) is a halogenated hydrocarbon.

4. A process according to any preceding claim, wherein said imidoyl halide starting reactant used in step (a) is prepared by reacting styrene with a halogen in the presence of a nitrile of the formula:

$$R_1—C\equiv N$$

wherein $R_1$ is as defined in Claim 1.

5. A process according to any preceding claim, wherein said imidoyl halide is reacted with methoxyethylamine in step (a).

**Patentansprüche**

1. Verfahren zur Herstellung von Tetramisol mit den folgenden Stufen:

(a) Umsetzung eines Imidoylhalogenids der Formel

$$X—C(R_1)=N—CH(Ar)—CH_2—X$$

wobei Ar für Phenyl steht, wobei $R_1$ für Wasserstoff, Niederalkyl oder Phenyl oder Niederalkyl-subst.-phenyl steht und wobei X für Halogen steht, mit einem Hydroxyäthylamin oder Alkoxyäthylamin der Formel

$$R_2O—(CH_2)_2—NH_2$$

wobei $R_2$ für Wasserstoff oder Niederalkyl steht, unter Gewinnung eines Amidinhydrohalogenids der Formel

$$R_2O—CH_2—CH_2—NH—C(R_1)=N—CH(Ar)—CH_2X.HX;$$

(b) Umsetzung des letzteren Amidinhydrohalogenids bei einer Temperatur im Bereich von —20 bis

50°C mit entweder einer anorganischen Base oder einem Hydroxyäthylamin oder Alkoxyäthylamin der Formel

$$H_2N—CH_2—CH_2—OR_2$$

in einem inerten Medium unter Gewinnung eines Imidazolins der Formel

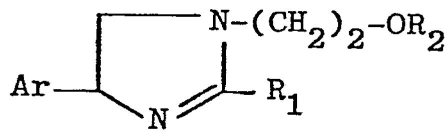

wobei Ar, $R_1$ und $R_2$ die oben angegebene Bedeutung haben;

(c) Hydrolyse des letzteren Imidazolins bei einer Temperatur im Bereich von 25 bis 150°C unter Spaltung des Rings und unter Gewinnung eines Amidoamins der Formel

$$R_1CONH—CH(Ar)—CH_2—NH—CH_2—CH_2—OR_2$$

wobei Ar, $R_1$ und $R_2$ die oben angegebene Bedeutung haben;

(d) weitere Umsetzung des letzteren Amidoamins mit einer zweckentsprechenden anorganischen Base oder Protonenquelle unter Gewinnung eines Diamins der Formel

$$NH_2—CH(Ar)—CH_2—NH—CH_2—CH_2—OR_2$$

wobei Ar, $R_1$ und $R_2$ die oben angegebene Bedeutung haben;

(e) Umsetzung des letzteren Diamins mit Schwefelkohlenstoff unter Gewinnung eines Dithiocarbamats der tautomeren Formel

$$^{\ominus}SC(S)—NH—CH(Ar)—CH_2—^{\oplus}NH_2(CH_2)_2—OR_2;$$

(f) Erhitzen des letzteren Dithiocarbamats unter Gewinnung eines Thions der Formel

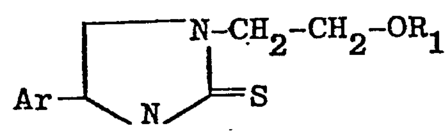

;

(g) Umsetzung dieses Thions mit einer Säure der Formel HA unter Gewinnung eines Tetramisolsalzes der Formel

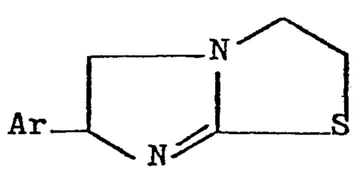

. HA

wobei Ar, $R_1$ und $R_2$ die oben angegebene Bedeutung haben und wobei A für ein Anion einer pharmazeutisch akzeptablen Säure steht; und, falls erwünscht,

(h) Neutralisierung des letzteren Salzes unter Gewinnung eines Tetramisols in Form der freien Base der Formel

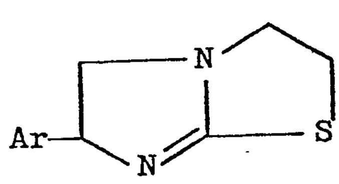

2. Verfahren nach Anspruch 1, dadurch gekennzeichnet, daß man die Stufe (a) durchführt unter Zusatz des Hydroxyäthylamins oder des Alkoxyäthylamins zum Imidoylhalogenid im Verlauf einer Zeitdauer von 1/2 bis 4 Stunden, wobei man die Temperatur während der Aminzugabe im Bereich von unterhalb 20°C hält, worauf man die Reaktion bei einer höheren Temperatur vervollständigt.

3. Verfahren nach Anspruch 1 oder Anspruch 2, wobei das inerte Medium der Stufe (b) ein halogenierter Kohlenwasserstoff ist.

4. Verfahren nach einem der vorhergehenden Ansprüche, dadurch gekennzeichnet, daß das Imidoylhalogenid, welches als Ausgangsreaktant der Stufe (a) eingesetzt wird, hergestellt wurde durch Umsetzung von Styrol mit einem Halogen in Gegenwart eines Nitrils der Formel

$$R_1—C{\equiv}N$$

wobei $R_1$ die in Anspruch 1 angegebene Bedeutung hat.

5. Verfahren nach einem der vorhergehenden Ansprüche, wobei das Imidoylhalogenid mit Methoxyäthylamin in Stufe (a) umgesetzt wird.

**Revendications**

1. Procédé de préparation de tétramisole qui comprend les stades consistant à:

(a) faire réagir un halogénure d'imidoyle répondant à la formule:

$$X—C(R_1)=N—CH(Ar)—CH_2—X$$

dans laquelle Ar représente le radical phényle, $R_1$ représente un atome d'hydrogène, un radical alkyle inférieur, phényle ou (alkyl inférieur)-phényle, et X représente un radical halogéno, avec l'hydroxyéthylamine ou une alcoxyéthylamine répondant à la formule

$$R_2O—(CH_2)_2—NH_2$$

dans laquelle $R_2$ représente un atome d'hydrogène ou un radical alkyle inférieur pour obtenir un halogénhydrate d'amidine répondant à la formule:

$$R_2O—CH_2—CH_2—NH—C(R_1)=N—CH(Ar)—CH_2X.HX;$$

(b) faire réagir ce dernier halogénhydrate d'amidine à une température comprise entre −20°C et 50°C avec une base minérale, l'hydroxyéthylamine ou une alcoxyéthylamine répondant à la formule

$$H_2N—CH_2—CH_2—OR_2$$

dans un milieu inerte pour obtenir une imidazoline répondant à la formule:

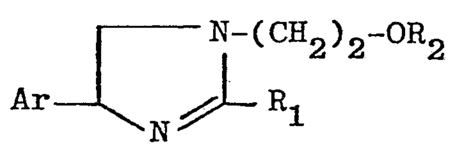

dans laquelle Ar, $R_1$ et $R_2$ ont la même définition que ci-dessus;

(c) hydrolyser cette dernière imidazoline à une température comprise entre 25°C et 150°C pour ouvrir le cycle et obtenir ainsi une amidoamine répondant à la formule:

$$R_1CONH—CH(Ar)—CH_2—NH—CH_2—CH_2—OR_2$$

dans laquelle, Ar, $R_1$ et $R_2$ ont la même définition que ci-dessus;

(d) faire ensuite réagir cette dernière amidoamine avec une base minérale appropriée ou une source protique pour obtenir une diamine répondant à la formule:

$$NH_2—CH(Ar)—CH_2—NH—CH_2—CH_2—OR_2$$

dans laquelle Ar, $R_1$ et $R_2$ ont la même définition que ci-dessus;

(e) faire réagir cette dernière diamine avec du disulfure de carbone pour obtenir un dithiocarbamate répondant à la formule tautomère:

$$^{\ominus}SC(S)—NH—CH(Ar)—CH_2—{}^{\oplus}NH_2(CH_2)_2—OR_2;$$

(f) chauffer ce dernier dithiocarbamate pour produire une thione répondant à la formule:

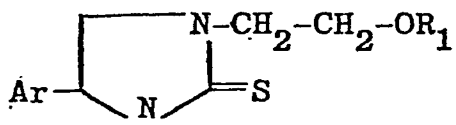

;

(g) faire réagir cette dernière thione avec un acide répondant à la formule HA pour obtenir un sel de tétramisole répondant à la formule:

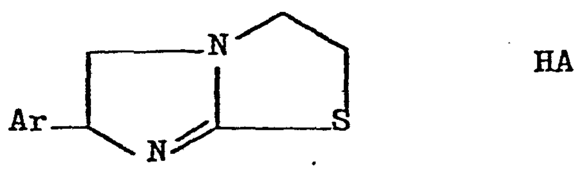

dans laquelle Ar, $R_1$ et $R_2$ ont la même définition que ci-dessus et A représente un anion d'un acide pharmaceutiquement acceptable, et, si on le désire,

(h) neutraliser ce dernier pour obtenir le tétramisole sous forme de la base libre répondant à la formule:

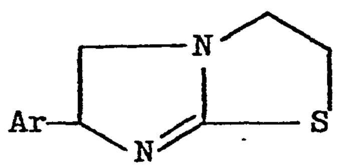

2. Procédé selon la revendication 1, dans lequel on effectue le stade (a) par addition de ladite hydroxyéthylamine ou de ladite alcoxyéthylamine audit halogénure d'imidoyle en une période de 0,5 à 4 heures en maintenant la température pendant cette addition de l'amine en dessous de 20°C, après quoi la réaction est achevée à une température plus élevée.

3. Procédé selon l'une des revendications 1 ou 2, dans lequel ledit milieu inerte utilisé dans le stade (b) est un hydrocarbure halogéné.

4. Procédé selon l'une quelconque des revendications précédentes dans lequel ledit halogénure d'imidoyle réagissant de départ utilisé dans le stade (a) est préparé par réaction du styrène avec un halogène en présence d'un nitrile de formule:

$$R_1—C{\equiv}N$$

dans laquelle $R_1$ a la même définition que dans la revendication 1.

5. Procédé selon l'une quelconque des revendications précédentes, dans lequel on fait réagir ledit halogénure d'imidoyle avec la méthoxyéthylamine dans le stade (a).